(19) Europäisches Patentamt
European Patent Office
Office européen des brevets

(11) Publication number: 0 089 900
B1

(12) # EUROPEAN PATENT SPECIFICATION

(45) Date of publication of the patent specification: 27.12.85

(51) Int. Cl.⁴: C 07 D 207/27, A 61 K 31/495

(21) Application number: 83400607.4

(22) Date of filing: 23.03.83

(54) **New N-((2-oxo-1-pyrrolidinyl)acetyl)piperazines, the methods of producing such new compounds and their salts as well as pharmaceutical preparations for therapeutic use containing these compounds or salts.**

(30) Priority: 24.03.82 ES 510759
05.04.82 ES 511181

(43) Date of publication of application:
28.09.83 Bulletin 83/39

(45) Publication of the grant of the patent:
27.12.85 Bulletin 85/52

(84) Designated Contracting States:
AT BE CH DE FR GB IT LI LU NL SE

(56) References cited:
EP - A - 0 005 689
FR - A - 2 368 275
GB - A - 1 039 113
GB - A - 1 309 692

CHEMICAL ABSTRACTS, vol. 94, no. 4, 26th January 1981, page 570, no. 30707u, Columbus Ohio (USA); S. ZIKOLOVA et al.: "Synthesis of piperazine derivatives. XIII. Preparation of NI-substituted N4-(chloroacetyl)piperazines"
CHEMICAL ABSTRACTS, vol. 87, 1977, page 576, no. 152011p, Columumbus Ohio (USA);
CHEMICAL ABSTRACTS, vol 98, 1983, page 562, no. 89386z, Columubus Ohio (USA);

(73) Proprietor: PRODES S.A., rue Trabajo s/n., San Justo Desvern (Barcelona) (ES)

(72) Inventor: Veit, Dagmar Maria Vedrilla, rue de la Marinada s/n., Sant Joan Despi (Barcelona) (ES)

(74) Representative: Chauchard, Robert et al, c/o Cabinet Malémont 42, avenue du Président Wilson, F-75116 Paris (FR)

EP 0 089 900 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to N-[(2-oxo-1-pyrrolidinyl)acetyl]-piperazines which are of use as medicines and as intermediates for the productions of medicines. This invention also relates to methods of producing these novel compounds. Similar compounds with similar activities have been disclosed in FR-A-2 368 275, GB-A-1 093 113, and GB-A-1 309 692.

More specifically, this invention relates to piperazines of the formula I

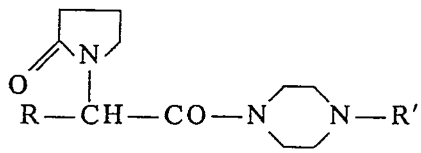

(I)

wherein

R represents hydrogen or an alkyl group which contains 1 to 3 carbon atoms which can be arranged as straight or branched chain and

R' represents an allyl, cinnamyl or p-methoxybenzyl group.

The following are included as biologically acceptable salts: acetates, citrates, succinates, maleates, asorbates, hydrochlorides, sulfates, etc. These salts may be prepared by known salification methods.

Preferred compounds in this invention are:

N-[(2-oxo-1-pyrrolidinyl)acetyl]-N'-allylpiperazine
N-[(2-oxo-1-pyrrolidinyl)acetyl]-N'-cinnamylpiperazine and
N-[(2-oxo-1-pyrrolidinyl)acetyl]-N'-(p-methoxybenzyl)piperazine.

The N-[(2-oxo-1-pyrrolidinyl)acetyl]piperazines of the present invention may be produced by means of the following methods:

I. By amminolysis of an ester, illustrated as follows:

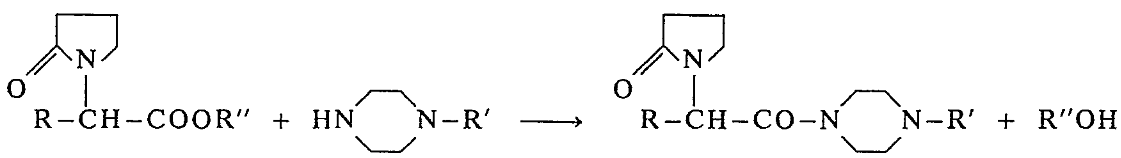

(II) (III) (I) (IV)

wherein R and R' have the same meanings as above and R" represents an alkyl radical which contains 1 or 2 carbon atoms or an aryl radical with or without substitution.

The method's characteristic is in the use of a stoichiometric quantity, or an excess (1 to 5 mol) or formula II ester in relation to amine III. The formula IV alcohol formed is gradually eliminated by distillation at normal or reduced pressure. In this last case a 14 mm vacuum is enough. The reaction's temperature depends on the type of ester used; in the case R" represents a substituted or non-substituted phenyl radical, the working temperature should be room temperature or slightly higher, for example 80°C. In the case R" represents alkyl radicals the working temperature should be between 100 and 190°C. Reaction times should be of 6 to 14 hours. Formula I compounds are isolated as free bases by distillation or crystallization. In the case when excess formula II ester is used, it is desirable to eliminate this ester by previous distillation in a vacuum. Formula II esters may be recycled in a different operation.

II. By alkylation of a salt of pyrrolidinone (preferentially the sodium or the potassium salt) with a haloacetamide of formula V illustrated as follows:

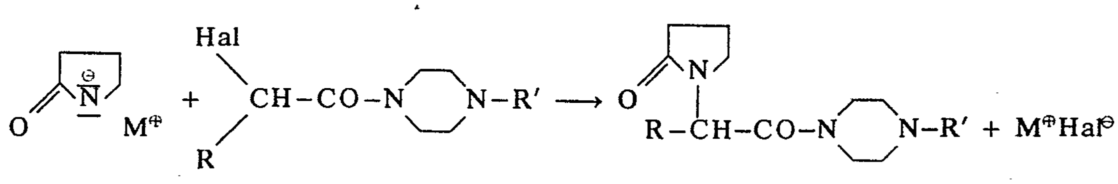

(V) (I)

wherein R and R' have the same meanings as above and Hal represents an hologen atom, preferentially chlorine or bromine.

The solvents employed are aromatic hydrocarbons, for instance benzene, toluene or xylenes. The reaction's temperature may oscillate between 75 and 130°C.

Formula V haloacetamides are prepared by reacting substituted piperazines of formula III

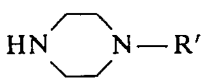

(III)

wherein R' has the same meaning as above with the acid chloride of a haloacetic acid of formula VI

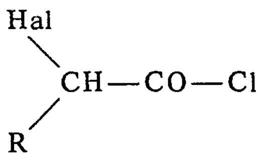

(VI)

illustrated as follows:

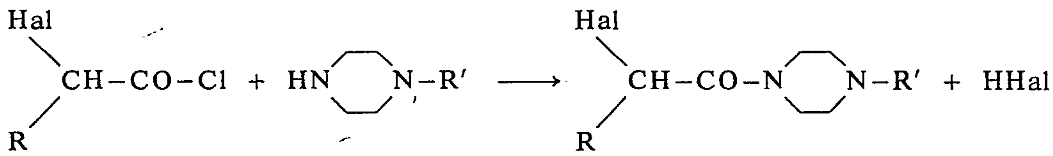

(V)

Inert solvents such as ether, benzene, toluene or xylene may be used as solvents. Most suitable working temperature is room temperature in presence of one mol of base, in order to neutralize the acid formed. Inorganic salts may be used as bases, for instance thoroughly grinded carbonates, or organic bases for instance triethylamine; preferentially an excess of the substituted piperazine should be used. The compound N-chloroacetyl-N'-cinnamyl piperazine is described by S. Zikolova et al.: Tr. Nauchnoizsled. Khim.-Farm. Inst. 10, 47 (CA 94 30707 u).

III. By acyclation of the substituted piperazines of the formula III with the acid chloride of a 2-(2-oxo-1-pyrrolidinyl). Acetic acid of the formula VII illustrated as follows:

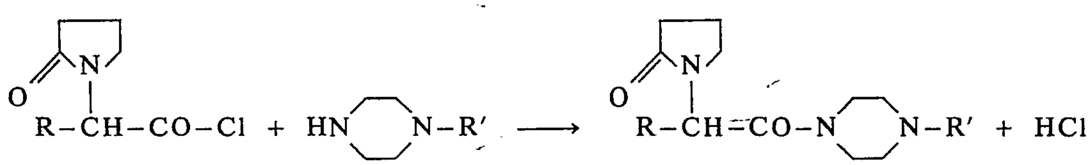

(VII) (III) (I)

The radicals R and R' have the same meaning as above.

The acid chlorides of formula VII are prepared from the 2-(2-oxo-1-pyrrolidinyl)acetic acids and $SOCl_2$.

Benzene or toluene may be used as solvents. It is also possible to work with an excess $SOCl_2$ as solvent. Working temperature should be room temperature. Appropriate time of reaction is between 2 and 3 hours. After elimination of solvent or excess $SOCl_2$, the acid chlorides of formula VII are used in crude state.

The reactions of compounds of formula VII with the substituted piperazines of formula III are performed by known methods in inert solvents such as ether, benzene or aliphatic hydrocarbons. Appropriate temperatures are between −10°C and +25°C. They may be performed in presence of an inorganic base, for instance carbonates, or of organic bases, for instance triethylamine, in order to neutralize the hydrochloric acid formed. It is also possible to use the substituted piperazines of formula III as base, thus working in the 1/2 = VII/III molar proportion. It is also possible to work in the 1/1 = VII/III molar proportion. In this last case, more polar solvents should be used, such as $CHCl_3$ or $CH_3CN$ and the compounds of formula I are obtained in their hydrochloride form.

The N-[(2-oxo-1-pyrrolidinyl)acetyl]piperazines, according to this invention are compounds which show pharmacological actions useful in therapeutical purposes.

The following results were obtained in experimental studies:

Electromagnetic blood flow measurement

As can be seen in Meth and Find. Exptl. Clin. Pharmacol. 3 (6) 397—401 (1981) — J. Roca, M. Grau and J. Balasch., the measurement of cerebral blood flow was performed by attaching an electromagnetic blood flow transducer to the vertebral artery of the anesthetized dogs.

After orotraqueal intubation, artificial respiration with normal air was instituted using a Manley Pulmovent respirator. The correct ventilation of the dogs was checked after all the surgical procedures by determining the $pO_2$, $pCO_2$ and pH in arterial blood samples, as well as several times during the experiment by means of an Astrup apparatus. Blood pressure (4) was measured in left saphenous artery by means of a Bell & Howell pressure transducer, and the heart rate by means of a Beckman ECG device.

Recordings were made on an ink-writing Beckman polygraph. Left saphenous vein and right saphenous artery were canulated with polyethylene catheters for drug administration and blood collection respectively.

Vertebral blood flow was measured by a non-cannulating electromagnetic flowmeter (Carolina Medical Electronics) placed in the vertebral artery.

The Carolina digital flowmeter was connected to an Omniscribe Houston Instruments plotter.

Piracetam and nicotinic acid were used as control drugs.

The results are shown in the following table:

Table I

| Control and synthetized drugs | effect | dose |
|---|---|---|
| (2-oxo-1-pyrrolidinyl)acetamide | No effect | |
| Nicotinic acid | No effect | |
| N-[(2-oxo-1-pyrrolidinyl)acetyl]-N'-(p-methoxybenzyl)piperazine | Active | 1 mg/kg |
| N-[(2-oxo-1-pyrrolidinyl)acetyl]-N'-cinnamylpiperazine | Active | 2,5 mg/kg |
| N-[(2-oxo-1-pyrrolidinyl)acetyl]-N'-allylpiperazine | Active | 2,5 mg/kg |

Non specific protection against il lethality by »nootropic« drugs

It is known that the nootropic drugs, as Piracetam, as well as the phenobarbital or hydantoin have a protection on the survival time of animals. When the subjects are submitted to several types of hypoxia, the lethality is significantly diminished by the »protection« given by the nootropic drug.

In the intention to increase the accuracy, we have a three trials panel in order to study the effect of the new synthetized drugs.

We have chosen the hypoxia-hypobara: the decompression kills the animals, because of the lack of $O_2$. The survival time is clearly increased by the active drugs.

The second trial is the quick decapitation of mouse. The head has a typical mandibular movement, known as gasping, as searching the breathing air. The gasping time is well prolonged by the active-drugs.

The third trial is by CNK. Obviously CNK kills the animals. Again the »protected« animals live a good number of seconds more than the controls.

The summary of results is found in Table II.

Table II

| Control and synthetized drugs | dose | hypoxia hypobara | gasping | anoxia by CNK |
|---|---|---|---|---|
| Pentobarbital | 40 mg/kg | Active | Active | Active |
| Diphenylhydantoine | 100 mg/kg | Active | Very Active | Active |
| (2-oxo-1-pyrrolidinyl)acetamide | 1000 mg/kg | No effect | No effect | No effect |

Continuation

| Control and synthetized drugs | dose | hypoxia hypobara | gasping | anoxia by CNK |
|---|---|---|---|---|
| N-[(2-oxo-1-pyrrolidinyl)acetyl]-N'-(p-methoxybenzyl)piperazine | 250 mg/kg | Active | Active | No effect |
| N-[(2-oxo-1-pyrrolidinyl)acetyl]-N'-cinnamylpiperazine | 300 mg/kg | Active | Active | Active |
| N-[(2-oxo-1-pyrrolidinyl)acetyl]-N'allylpiperazine | 500 mg/kg | Active | Active | Active |

$LD_{50}$ of the three representative compounds are included in Table III:

Table III

| Compound | $LD_{50}$ |
|---|---|
| N-[(2-oxo-1-pyrrolidinyl)acetyl]-N'-(p-methoxybenzyl)piperazine | 1,112 mg/kg |
| N-[(2-oxo-1-pyrrolidinyl)acetyl]-N'-cinnamylpiperazine | 1,400 mg/kg |
| N-[(2-oxo-1-pyrrolidinyl)acetyl]-N'-allylpiperazine | 4,000 mg/kg |

In view of the above pharmacological and toxicological results, it appears that the compounds of formula (I) may be used as drugs in therapeutics, especially in the treatment of cerebrovascular disturbances in the elderly.

The present invention extends therefore to pharmaceutical compositions containing (a) as active ingredient at least one of the compounds of formula (I) and (b) a pharmaceutically acceptable carrier, these compositions being preferably formulated with a view to their oral administration. Thus they may be administrated orally for example in the form of pills, capsules or tablets, in an amount of 10 to 100 mg of active ingredient (twice a day).

The following examples will further illustrate the invention without, however, limiting it thereto.

## Example 1

According to method I, 33,4 g (165 mmoles) of N-cinnamylpiperazine are mixed with 56,5 g (330 mmoles) of ethyl 2-(2-oxo-1-pyrrolidinyl-acetate. In a 14 mm vacuum this mixture is heated to 180°C during 7 hours. After this heating period, the excess ester is distilled in a vacuum. 25 ml ethyl acetate are added and the crystals formed filtered. By crystallization of the same of 70 ml ethyl acetate 51,2 g (156 mmoles; 95%) of N-[(2-oxo-1-pyrrolidinyl)-acetyl]-N'-cinnamylpiperazine are obtained in the form of white crystals having a melting point of 113°–5°C.

| | | | |
|---|---|---|---|
| Calc. ($C_{19}H_{25}N_3O_2$): | 69,70% C | 7,70% H | 12,83% N |
| Found: | 69,85% C | 7,81% H | 12,70% N |

$R_f$ = 0,45 ($CHCl_3$/MeOH = 100/15)

The IR spectrum (KBr) shows, among others, the following bands:

3820, 2770, 1680, 1660, 1600, 1585, 995, 965, 790, 740 and 690 $cm^{-1}$.

Example 2

According to method I 68,87 g (550 mmoles)) of N-allyl-piperazine are mixed with 190,09 g (1,11 moles) of ethyl 2-(2-oxo-1-pyrrolidinyl)acetate.

The mixture is heated to 175—180°C during 7 hours. After this period of time, the temperature is increased to 190°C and the mixture kept at this temperature for two more hours. At the same time, the ethanol formed by distillation at normal pressure is eliminated and 12,6 g are collected. Once the heating period over, the mixture is distilled in a vacuum and 105,69 g (420 mmoles; 76%) of N-[(2-oxo-1-pyrrolidinyl)acetyl]-N'-allylpiperazine collected in the form of a heavy oil with a boiling point of 200—202°C/0,5 mm.

| | | | |
|---|---|---|---|
| Calc. ($C_{13}H_{21}N_3O_2$): | 62,12% C | 8,42% H | 16,72% N |
| Found: | 62,30% C | 8,57% H | 16,60% N |

The IR spectrum ($CHCl_3$) shows among others the following bands:

1680, 1655, 1465, 1455, 1290 and 100 $cm^{-1}$.

From 14,176 g (56,5 mmoles) of N-[(2-oxo-1-pyrrolidinyl)acetyl]-N'-allypiperazine and 6,547 g (56,5 mmoles) of maleic acid, dissolved in MeOH, 17,245 g (46,93 mmoles; 83%) of the piperazine maleate mentioned at the beginning are obtained, with a melting point of 163—5°C (EtOH).

| | | | |
|---|---|---|---|
| Calc. ($C_{17}H_{25}N_3O_6$): | 55,57% C | 6,86% H | 11,43% N |
| Found: | 55,69% C | 6,97% H | 11,27% N |

$R_f = 0,35$ (MeOH)

Example 3

According to method I the following products are mixed: 53,59 g (260 mmoles) of N-(p-methoxybenzyl)piperazine with 88,8 g (519 mmoles) of ethyl 2-(2-oxo-1-pyrrolidinyl)acetate.

In a 14 mm vacuum the mixture is heated to 175—80°C during 7 hours. Once the heating period over, the excess ester is distilled in a vacuum. The remaining product ist grinded with a mixture of 25 ml ethyl acetate and 150 ml ether. By filtration the following ist obtained: 72,69 g (219 mmoles; 85%) of N-[(2-oxo-1-pyrrolidinyl)acetyl]-N'-(p-methoxybenzyl)piperazine in the form of white crystals with a melting point of 87—9°C.

| | | | |
|---|---|---|---|
| Calc. ($C_{18}H_{25}N_3O_3$): | 65,23% C | 7,60% H | 12,68% N |
| Found: | 65,10% C | 7,75% H | 12,82% N |

$R_f = 0,51$ ($CHCl_3$/MeOH = 80/20)

The IR spectrum (KBr) shows, between others the following bands:

2800, 2780, 1680, 1660, 1610, 1585, 1290, 1280, 1240, 1030, 800 and 750 $cm^{-1}$.

Example 4

According to method I, the following products are mixed: 199,25 g (1 mol) of ethyl 2-(2-oxo-1-pyrrolidinyl)butyrate (previously prepared by alkylation of the sodium salt of pyrrolidinone with ethyl 2-bromobutyrate) and 101,15 g (0,5 mole) of N-cinnamyl-piperazine. In a 14 mm vacuum the mixture is heated to 175—80°C during 12 hours. Once the heating time over, the excess ester is distilled. The remaining product is grinded with ligroin (boiling point 80—100°C) and by filtration 158,69 g (446 mmoles; 89%) of N-[2-(2-oxo-1-pyrrolidinyl)butyroyl]-N'-cinnamyl-piperazine is obtained in the form of white crystals.

| | | | |
|---|---|---|---|
| Calc. ($C_{21}H_{29}N_3O_2$): | 70,95% C | 8,22% H | 11,82% N |
| Found: | 71,11% C | 8,34% H | 11,70% N |

## Example 5

According to method I, 199,25 g (1 mol) of ethyl 2-(2-oxo-1-pyrrolidinyl)butyrate are mixed with 103,146 g (500 mmoles) of N-(p-methoxybenzyl)piperazine and heated as described in Example 4. The excess ester is then eliminated by distillation in a vacuum. The remaining product is distributed between HCl IN an ether. The aequous phase ist extracted repeatedly with ether, NaOH is then added to the aequous phase until an alkaline pH is obtained, and evaporated to dryness. The remaining product is grinded with ether. By evaporating the ether 133,00 g (370 mmoles; 74%) of N-[2-(2-oxo-1-pyrrolidinyl)butyrol]-N'-(p-methoxybenzyl)-piperazine are obtained in the form of a heavy oil.

| | | | |
|---|---|---|---|
| Calc. ($C_{20}H_{29}N_3O_3$): | 66,82% C | 8,13% H | 11,69% N |
| Found: | 66,95% C | 8,26% H | 11,54% N |

## Example 6

According to method II, dissolve 28,7 g (141,8 mmoles) of N-cinnamyl-piperazine in 250 ml anhydrous toluene; the solution is then cooled to 0°C. A solution of 13,149 g (70,9 mmoles) of 2-bromobutyroyl-chloride (O. S. 30, 62, 1950) in 80 ml of toluene is then added drop by drop. Once all the amount of solution added, the mixture is stirred during 2 hours at room temperature. 16,3 g of the hydrochloride of N-cinnamylpiperazine is then obtained by filtration. The filtrate is evaporated to dryness, then dissolved in EtOH and acidified with EtOH/HCl. By precipitation with ether 22,5 g (58,0 mmoles; 82%) of the hydrochloride of N-cinnamyl-N'-(2-bromobutyroyl)piperazine are obtained in the form of white crystals with a melting point of 184—6°C (dec.).

| | | | |
|---|---|---|---|
| Calc. ($C_{17}H_{24}BrClN_2O$): | 52,65% C | 6,23% H | 7,22% N |
| Found: | 52,57% C | 6,10% H | 7,30% N |

The free base consists in a heavy oil. Its IR ($CCl_4$) shows, among others, the following bands:

2940, 2820, 2780, 1665, 1460, 1350, 1235, 1190, 1145, 1130, 1000, 970 and 690 $cm^{-1}$.

## Example 7

The sodium salt of pyrrolidinone is prepared with 3,8809 g (45,6 mmoles) of pyrrolidinone, 0,9614 g (41,8 mmoles) of Na and 100 ml toluene. At a temperature of 97°C we gradually add a solution of N-cinnamyl-N'-(2-bromobutyroyl)-piperazine previously prepared by neutralization with NaOH and toluene extraction of 14,737 g (38 mmoles) of the hydrochloride. Once all the solution added, the mixture is reflux boiled during 6,5 hours. The mixture is filtered, and the filtered product evaporated to dryness.

By crystallization of petroleum ether (boiling point 60—90°C) we obtain N-[2-(2-oxo-1-pyrrolidinyl)-butyroyl]-N'-cinnamylpiperazine.

The IR spectrum shows absorptions at 1690 and 1650 $cm^{-1}$.

## Example 8

According to method III, 100 ml $SOCl_2$ recently distilled are poured on 17,12 g (100 mmoles) of 2-(2-oxo-1-pyrrolidinyl)-butyric acid (CA 89 P 59834 r) and stirred at room temperature during 2 hours.

The excess $SOCl_2$ is then distilled by vacuum distillation. The residual oil obtained is dissolved in 100 ml toluene and a solution of 40,46 g (200 mmoles) of N-cinnamylpiperazine in 150 ml toluene is then added, drop by drop.

The mixture is filtered, and the filtered product evaporated to dryness, thus obtaining 32,35 g (91 mmoles; 91%) of N-[2-ethyl-2-(2-oxo-1-pyrrolidinyl)acetyl]-N'-cinnamylpiperazine.

## Examples of preparations ready for administration

When the new compounds of this invention are intended for use in the treatment of cerebrovascular disturbances in the elderly, typus of suitable preparations can be examplified as follows:

1. Tablet

| | | |
|---|---|---|
| (1) | N-[(2-oxo-1-pyrrolidinyl)acetyl]-N'-cinnamylpiperazine | 50 g |
| (2) | Lactose | 50 g |
| (3) | Corn starch | 29 g |
| (4) | Magnesium stearate | 1 g |
| | 1000 tablets | 130 g |

Components (1) and (2) and 17 g of the corn-starch (3) were granulated together with a paste prepared from 7 g of the corn-starch. To these granules were added the remaining 5 g of the corn-starch and component (4). The mixture was then compressed by a tabletting machine to prepare 1000 tablets of 7 mm. diameter, each containing 50 mg of (1).

2. Capsule

| | | |
|---|---|---|
| (1) | N-[(2-oxo-1-pyrrolidinyl)acetyl]-N'-allylpiperazine | 50 g |
| (2) | Lactose | 100 g |
| (3) | Cellulose fine powder | 45 g |
| (4) | Magnesium stearate | 5 g |
| | 1000 capsules | 200 g |

All the materials were mixed and filled into 1000 capsules of gelatin; each capsule contains 50 mg of (1).

**Claims for the Contracting States BE CH DE FR GB IT LI LU NL SE:**

1. N-[(2-oxo-1-pyrrolidinyl)acetyl]piperazines of the following formula:

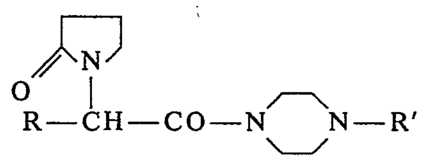

wherein

R represents hydrogen or an alkyl group which contains 1 to 3 carbon atoms which can be arranged as straight or branched chain and
R' represents an allyl, cinnamyl or p-methoxybenzyl group,

as well as their pharmaceutically acceptable acid addition salts.

2. N-[(2-oxo-1-pyrrolidinyl)acetyl]-N'-allylpiperazine
3. N-[(2-oxo-1-pyrrolidinyl)acetyl]-N'-cinnamylpiperazine
4. N-[(2-oxo-1-pyrrolidinyl)acetyl]-N'-(p-methoxybenzyl)piperazine.
5. A pharmaceutical composition which comprises:

a) as an active ingredient an effective amount of the piperazine compound as defined in claim 1) and
b) a pharmaceutically acceptable carrier or diluent therefor.

6. A method for producing a piperazine compound as defined in claim 1) consisting in subjecting a compound of the formula

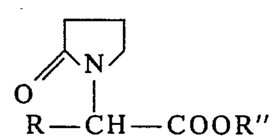

wherein R is as defined in claim 1 and R″ represents an alkyl radical which contains 1 or 2 carbon atoms or an aryl radical with or without substitution,
to amminolysis with a piperazine of the formula

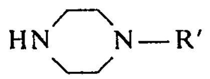

wherein R′ is as defined in claim 1.

7. Process for producing a piperazine compound as defined in claim 1, consisting in the alkylation of a salt of pyrrolidinone with a haloacetamide illustrated in the following sheet:

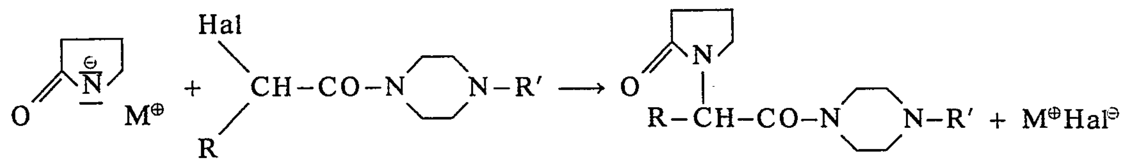

wherein R and R′ are as defined in claim 1.

8. Process according to claim 7, wherein the salt of pyrrolidinone is the sodium or the potassium salt.

9. Process for producing a piperazine compound as defined in claim 1, consisting in acyclating the substituted piperazines with an 2-(2-oxo-1-pyrrolidinyl)acetyl chloride illustrated in the following sheet:

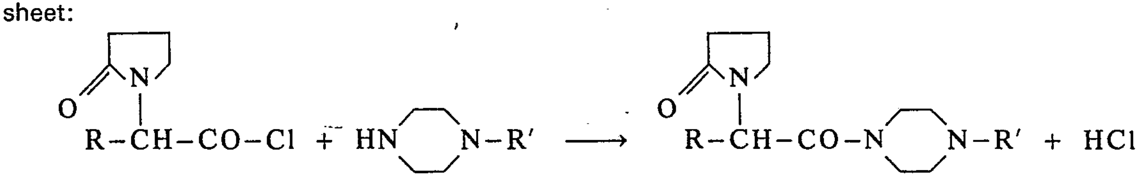

wherein R and R′ are as defined in claim 1.

**Claims for the Contracting State AT:**

1. Process for preparing a N-[(2-oxo-1-pyrrolidinyl)acetyl]piperazine of the following formula:

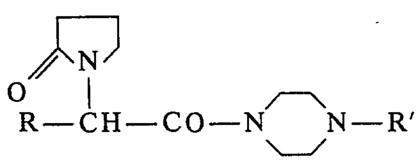

wherein

R represents hydrogen or an alkyl group which contains 1 to 3 carbon atoms which can be arranged as straight or branched chain, and
R′ represents an allyl, cinnamyl or p-methoxybenzyl group,

characterized in that it consists in subjecting a compound of the formula

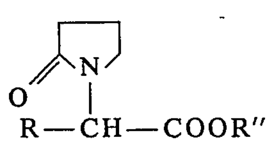

wherein R is as defined above and R″ represents an alkyl radical which contains 1 or 2 carbon atoms or an aryl radical with or without substitution,
to amminolysis with a piperazine of the formula

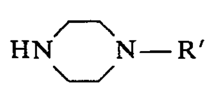

wherein R′ is as defined above.

2. A process for preparing a N-[(2-oxo-1-pyrrolidinyl)acetyl]piperazine of the following formula:

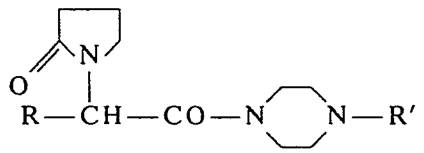

wherein

R represents hydrogen or an alkyl group which contains 1 to 3 carbon atoms which can be arranged as straight or branched chain, and
R' represents an allyl, cinnamyl or p-methoxybenzyl group,

characterized in that it consists in the alkylation of a salt of pyrrolidinone with a haloacetamide illustrated in the following sheet

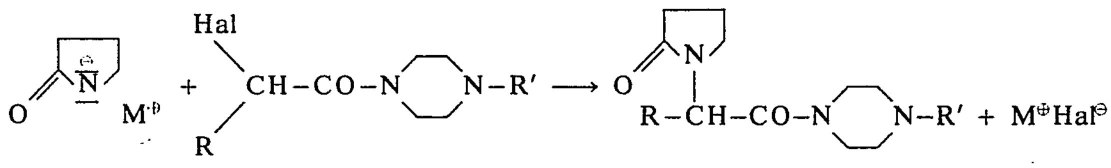

wherein R and R' are as defined in above.

3. Process according to claim 2, characterized in that the salt of pyrrolidinone is the sodium or the potassium salt.

4. A process for preparing a N-[(2-oxo-1-pyrrolidinyl)acetyl]piperazine of the following formula:

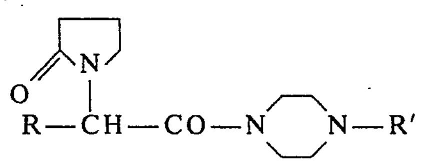

wherein

R represents hydrogen or an alkyl group which contains 1 to 3 carbon atoms which can be arranged as straight or branched chain, and
R' represents an allyl, cinnamyl or p-methoxybenzyl group,

characterized in that it consists in acyclating a substituted piperazine with an 2-(2-oxo-1-pyrrolidinyl)-acetyl choride illustrated in the following sheet:

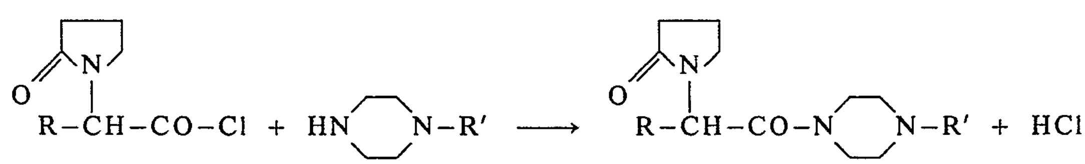

wherein R and R' are as defined above.

**Patentansprüche für die Vertragsstaaten BE CH DE FR GB IT LI LU NL SE:**

1. N-[(2-Oxo-1-pyrrolidinyl)acetyl]piperazine der folgenden Formel

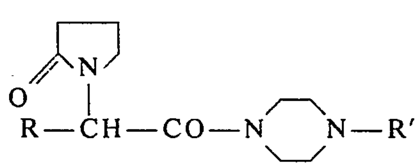

worin

R Wasserstoff oder eine Alkygruppe bedeutet, die 1 bis 3 Kohlenstoffatome enthält, welche als gerade oder verzweigte Kette angeordnet sein können, und
R' eine Allyl-, Cinnamyl- oder p-Methoxybenzylgruppe bedeutet,

sowie deren pharmazeutisch akzeptable Säureadditionssalze.
2. N-[(2-Oxo-1-pyrrolidinyl)acetyl]-N'-allylpiperazin.
3. N-[(2-Oxo-1-pyrrolidinyl)acetyl]-N'-cinnamylpiperazin.
4. N-[(2-Oxo-1-pyrrolidinyl)acetyl]-N'-(p-methoxybenzyl)piperazin.
5. Pharmazeutische Zusammensetzung, die

a) als aktiven Bestandteil eine wirksame Menge der Piperazinverbindung, wie in Anspruch 1 definiert, und
b) einen pharmazeutisch akzeptablen Träger oder Verdünner dafür

umfaßt.
6. Verfahren zur Herstellung einer Piperazinverbindung, wie in Anspruch 1 definiert, bestehend im Unterwerfen einer Verbindung der Formel

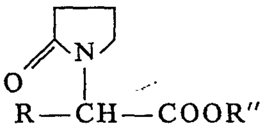

worin R wie in Anspruch 1 definiert ist und R'' einen Alkylrest, der 1 oder 2 Kohlenstoffatome enthält, oder einen Arylrest mit oder ohne Substitution bedeutet,
der Aminolyse mit einem Piperazin der Formel

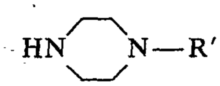

worin R' wie in Anspruch 1 definiert ist.
7. Verfahren zur Herstellung einer Piperazinverbindung, wie in Anspruch 1 definiert, bestehend im Alkylieren eines Salzes von Pyrrolidinon mit einem Halogenacetamid, veranschaulicht im folgenden Formelschema:

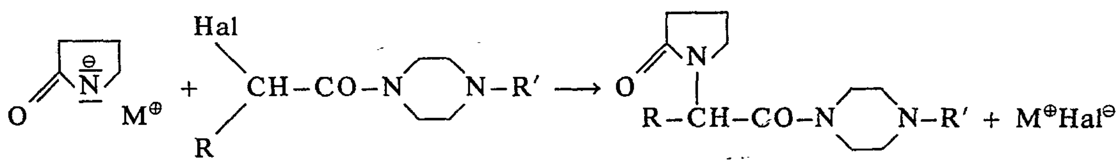

worin R und R' wie in Anspruch 1 definiert sind.
8. Verfahren nach Anspruch 7, worin das Salz von Pyrrolidinon das Natrium- oder das Kaliumsalz ist.
9. Verfahren zur Herstellung einer Piperazin-Verbindung, wie in Anspruch 1 definiert, bestehend im Acylieren der substituierten Piperazine mit 2-(2-Oxo-1-pyrrolidinyl)-acetylchlorid, veranschaulicht im folgenden Reaktionsschema:

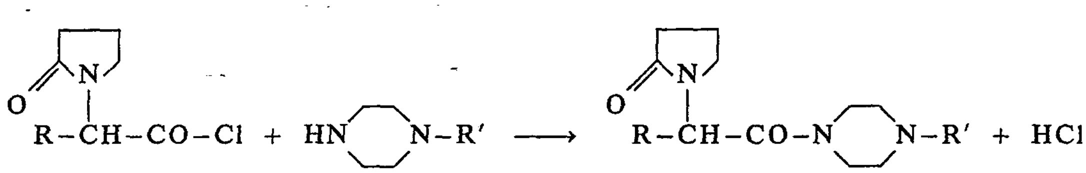

worin R und R' wie in Anspruch 1 definiert sind.

**Patentansprüche für den Vertragsstaat AT**

1. Verfahren zur Herstellung einer N-[(2-Oxo-1-pyrrolidinyl)acetyl]piperazine der folgenden Formel

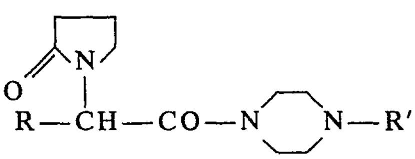

worin

R Wasserstoff oder eine Alkylgruppe bedeutet, die 1 bis 3 Kohlenstoffatome enthält, welche als gerade oder verzweigte Kette angeordnet sein können, und
R' eine Allyl-, Cinnamyl- oder p-Methoxybenzylgruppe bedeutet,

bestehend im Unterwerfen einer Verbindung der Formel

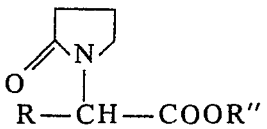

worin R wie oben definiert ist und R'' einen Alkylrest, der 1 oder 2 Kohlenstoffatome enthält, oder einen Arylrest mit oder ohne Substitution bedeutet, der Aminolyse mit einem Piperazin der Formel

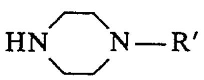

worin R' wie oben definiert ist.

2. Verfahren zur Herstellung einer N-[(2-Oxo-1-pyrrolidinyl)acetyl]piperazine der folgenden Formel

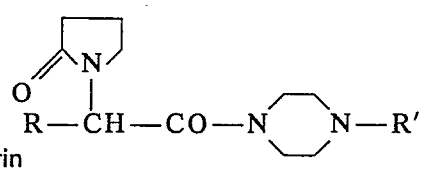

worin

R Wasserstoff oder eine Alkylgruppe bedeutet, die 1 bis 3 Kohlenstoffatome enthält, welche als gerade oder verzweigte Kette angeordnet sein können, und
R' eine Allyl-, Cinnamyl- oder p-Methoxybenzylgruppe bedeutet,

bestehend im Alkylieren eines Salzes von Pyrrolidinon mit einem Halogenacetamid, veranschaulicht im folgenden Formelschema:

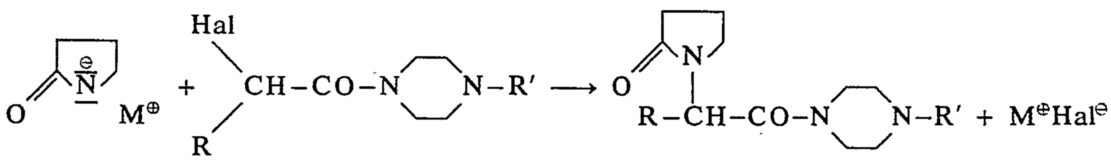

worin R und R' wie oben definiert sind.

3. Verfahren nach Anspruch 2, worin das Salz von Pyrrolidinon das Natrium- oder das Kaliumsalz ist.

4. Verfahren zur Herstellung einer N-[(2-Oxo-1-pyrrolidino)acetyl]piperazine der folgenden Formel

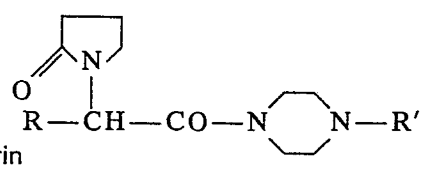

worin

R Wasserstoff oder eine Alkylgruppe bedeutet, die 1 bis 3 Kohlenstoffatome enthält, welche als gerade oder verzweigte Kette angeordnet sein können, und
R' eine Allyl-, Cinnamyl- oder p-Methoxybenzylgruppe bedeutet,

bestehend im Acylieren der substituierten Piperazine mit 2-(2-Oxo-1-pyrrolidinyl)-acetylchlorid, veranschaulicht im folgenden Reaktionsschema:

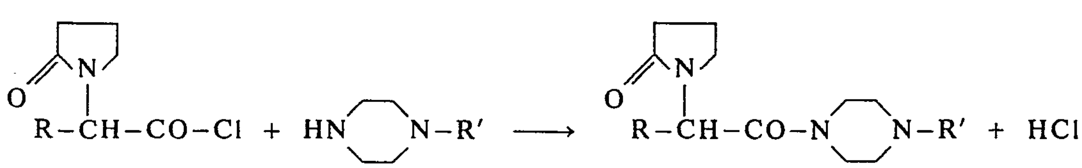

worin R and R' wie oben definiert sind.

**Revendications pour les Etats contractants BE CH DE FR GB IT LI LU NL SE:**

1. N-[(2-oxo-1-pyrrolidinyl)acétyl]pipérazines répondant à la formule suivante:

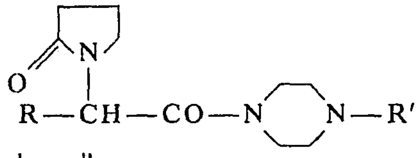

dans laquelle

R représente l'hydrogène ou un groupe alkyle qui contient 1 à 3 atomes de carbone et peut être choisi à chaîne droite ou ramifiée et
R' représente un groupe allyle, cinnamyle ou p-méthoxybenzyle,

ainsi que leurs sels d'addition avec un acide pharmaceutiquement acceptables.
2. La N'-[(2-oxo-1-pyrrolidinyl)acétyl]-N'-allylpipérazine
3. La N-[(2-oxo-1-pyrrolidinyl)acétyl]-N'-cinnamylpipérazine
4. La N-[(2-oxo-1-pyrrolidinyl)acétyl]-N'-(p-méthoxybenzyl)piperazine.
5. Composition pharmaceutique caractérisée en ce qu'elle comprend:

a) à titre d'ingrédient actif, une quantité efficace du composé constitué par la pipérazine telle que définie dans la revendication 1) et
b) un diluant ou véhicule pharmaceutiquement acceptable pour cela.

6. Procédé de préparation d'un composé constitué par la pipérazine telle que définie dans la revendication 1 caractérisé en ce qu'il consiste à soumettre un composé de formule

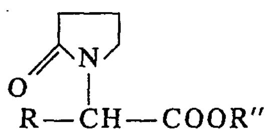

dans laquelle R est tel que défini dans la revendication 1 et R" représente un radical alkyle qui contient 1 ou 2 atomes de carbone ou un radical aryle avec sans substitution,
à une aminolyse avec une pipérazine de formule

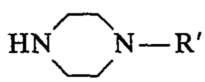

dans laquelle R' est tel que défini dans la revendication 1.
7. Procédé de préparation d'un composé constitué par la pipérazine telle que définie dans la revendication 1, caractérisé en ce qu'il comprend l'alcoylation d'un sel de la pyrrolidinone avec un haloacétamide illustrée dans le schéma suivant:

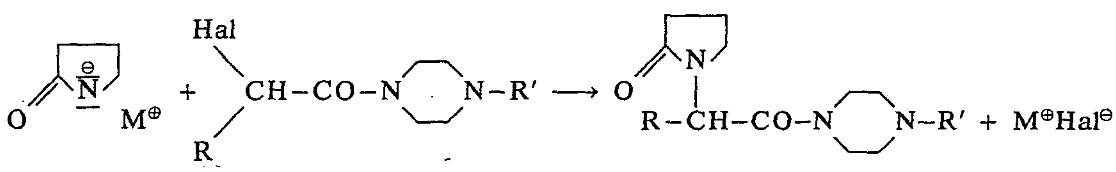

dans lequel R et R' sont tels que définis dans la revendication 1.
8. Procédé selon la revendication 7, caractérisé en ce que le sel de la pyrrolidinone est le sel de sodium ou le sel de potassium.
9. Procédé de préparation d'un composé constitué par la pipérazine telle que définie dans la revendication 1 caractérisé en ce qu'il consiste à acyler les pipérazines substituées avec un chlorure de 2-(2-oxo-1-pyrrolidinyl)acétyle comme illustré dans le schéma suivant:

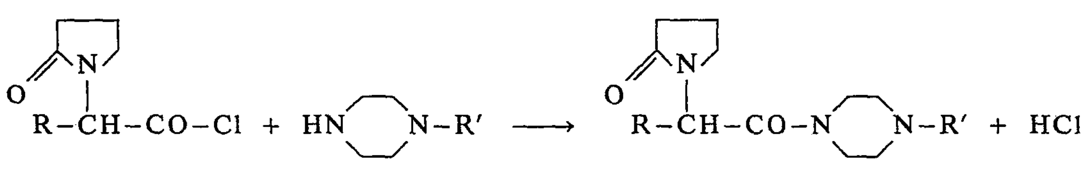

dans lequel R et R' tels que définis dans la rendication 1.

**Revendications pour l'Etat contractant AT:**

1. Procédé de préparation de N-[(2-oxo-1-pyrrolidinyl)acétyl] pipérazines répondant à la formule suivante:

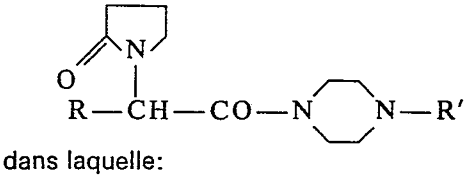

dans laquelle:

R représente l'hydrogène ou un groupe alkyle qui contient 1 à 3 atomes de carbone et peut être choisi à chaîne droite ou ramifiée et

R' représente un groupe allyle, cinnamyle ou p-méthoxybenzyle, caractérisé en ce qu'il consiste à soumettre un composé de formule:

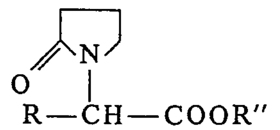

dans laquelle R est tel que défini ci-dessus et R" représente un radical alkyle qui contient 1 ou 2 atomes de carbone ou un radical aryle avec ou sans substitution,
à une aminolyse avec une pipéranzine de formule:

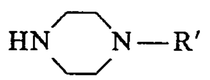

dans laquelle R' est tel que défini ci-dessus.

2. Procédé de préparation de N-[(2-oxo-1-pyrrolidinyl)acétyl] pipérazines répondant à la formule suivante:

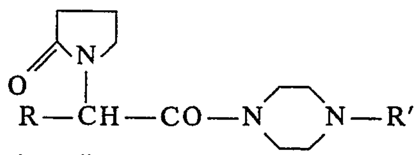

dans laquelle:

R représente l'hydrogène ou un groupe alkyle qui contient 1 à 3 atomes de carbone et peut être choisi à chaîne droite ou ramifiée et

R' représente un groupe allyle, cinnamyle ou p-méthoxybenzyle,

caractérisé en ce qu'il comprend l'alcoylation d'un sel de la pyrrolidinone avec un haloacétamide illustrée dans le schéma suivant:

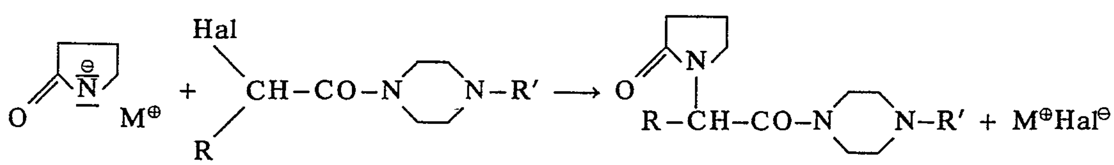

dans lequel R et R' sont tels que definis ci-dessus.

3. Procédé selon la revendication 2, caractérisé en ce que le sel de la pyrrolidinone est le sel de sodium ou le sel de potassium.

4. Procédé de préparation de N-[(2-oxo-1-pyrrolidinyl)acétyl]pipérazines répondant à la formule suivante:

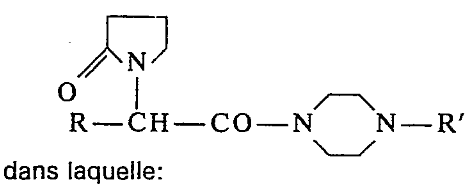

dans laquelle:

R représente l'hydrogène ou un groupe alkyle qui contient 1 à 3 atomes de carbones et peut être choisi à chaîne droite ou ramifiée et

R' représente un groupe allyle, cinnamyle ou p-méthoxybenzyle,

caractérisé en ce qu'il consiste à acyler des pipérazines substituées avec un chlorure de 2-(2-oxo-1-pyrrolidinyl)acétyle comme illustré dans le schéma suivant:

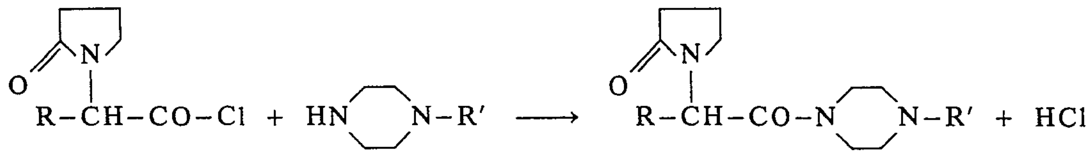

dans lequel R et R' sont tels que définis ci-dessus.